(19) **Europäisches Patentamt**
**European Patent Office**
**Office européen des brevets**

(11) **EP 2 905 612 B1**

(12) **EUROPEAN PATENT SPECIFICATION**

(45) Date of publication and mention
of the grant of the patent:
**01.03.2017 Bulletin 2017/09**

(51) Int Cl.:
*G01N 17/02* *(2006.01)*    *G01N 33/20* *(2006.01)*

(21) Application number: **12886067.3**

(86) International application number:
**PCT/JP2012/076256**

(22) Date of filing: **03.10.2012**

(87) International publication number:
**WO 2014/054186 (10.04.2014 Gazette 2014/15)**

(54) **APPARATUS FOR MEASURING AMOUNT OF HYDROGEN PENETRATED INTO METAL**

VORRICHTUNG ZUR MESSUNG DER MENGE VON IN EIN METALL EINGEDRUNGENEM WASSERSTOFF

DISPOSITIF PERMETTANT DE DÉTERMINER LA QUANTITÉ D'HYDROGÈNE PÉNÉTRANT DANS UN MÉTAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**12.08.2015 Bulletin 2015/33**

(73) Proprietors:
• **JFE Steel Corporation**
**Tokyo 100-0011 (JP)**
• **Tokyo Institute of Technology**
**Tokyo 152-8550 (JP)**

(72) Inventors:
• **OOTSUKA, Shinji**
**Tokyo 100-0011 (JP)**
• **NAKAMARU, Hiroki**
**Tokyo 100-0011 (JP)**
• **FUJITA, Sakae**
**Tokyo 100-0011 (JP)**
• **TSURU, Tooru**
**Tokyo 152-8552 (JP)**

(74) Representative: **Grünecker Patent- und
Rechtsanwälte
PartG mbB
Leopoldstraße 4
80802 München (DE)**

(56) References cited:
**CN-A- 101 832 966    JP-A- S57 119 254
JP-A- 2011 179 893    JP-A- 2011 179 893
US-A- 3 415 685**

• **SHINJI OTSUKA ET AL.: 'Jidosha Soko Kankyo
ni Okeru Kozai eno Suiso Shinnyuryo no Renzoku
Monitoring Gijutsu no Kaihatsu' CURRENT
ADVANCES IN MATERIALS AND PROCESSES
vol. 25, 01 March 2012, page 341, XP008176285**

**Description**

TECHNICAL FIELD

The present invention relates to an apparatus for measuring an amount of hydrogen penetrated into metal, which is capable of accurately measuring an amount of hydrogen penetrating into metal due to the corrosion of the metal.

BACKGROUND ART

In recent years, in view of preventing global warming, there are demands for improving energy efficiency by reducing weight of moving bodies such as automobiles, marine vessels, and train cars. In the case of automobiles, in particular, improvement in fuel efficiency of gasoline is required. In this regard, materials are also required to be reduced in weight, and in particular, high-strengthened steel sheets are in greater use as steel materials, in order to ensure equal safety even when the sheet thickness is reduced.

However, it is known that a steel material increased in strength becomes more susceptible to a "delayed fracture" phenomenon. The "delayed fracture" is significantly caused along with the increase in strength of the steel material, and becomes particularly noticeable in high-strength steel having a tensile strength of equal to or larger than 1,180 MPa (Non-patent Literature 1). Here, the "delayed fracture" is a phenomenon that generates sudden brittle fractures in a high strength steel sheet under static load (load stress equal to or smaller than the tensile strength) applied for a certain period of time, without exhibiting hardly any plastic deformation in appearance. The delayed fracture herein particularly refers to hydrogen embrittlement induced by hydrogen penetrating into the steel material.

Hydrogen penetrating into a steel material is considered to be part of hydrogen generated along with the corrosion of the steel sheet. With this in view, there have been proposed various methods of evaluating the delayed fracture focusing on hydrogen penetrating into the steel material.

For example, Patent Literature 1 proposes an evaluation method of delayed fracture characteristics of a steel material by adding diffusible hydrogen to the steel material through cathodic charge so as to measure the limit amount of diffusible hydrogen, in which the steel material is galvanized in order to prevent hydrogen from being discharged from the steel material during the measurement of the limit amount of diffusible hydrogen.

However, the technology disclosed in Patent Literature 1 employs an accelerated test which causes hydrogen to be forced to penetrate into the steel through cathodic charge, and therefore only capable of determining the order of superiority, in terms of susceptibility to delayed fracture, depending on the type of the sample material under conditions different from those of an actual use environment. Accordingly, there cannot be obtained information for determining whether or not an amount of hydrogen that penetrates due to corrosion under an actual use environment causes delayed fracture.

Further, in recent years, various studies have been reported on the hydrogen penetration. For example, Non-patent Literature 2 reports on the behavior of hydrogen penetration evaluated with the use of ammonium thiocyanate. Non-patent Literature 2 compares the hydrogen penetration observed by using ammonium thiocyanate with the hydrogen penetration caused by cathodic charge.

However, the amount of hydrogen penetration obtained by the evaluation method with the use of ammonium thiocyanate as disclosed in Non-patent Literature 2 cannot be considered to be equal to the amount caused by surface corrosion, and thus, it cannot be measured how the behavior of hydrogen penetration is influenced by, for example, zinc coating or the like which is used for the purpose of anti-rust in the field of automobiles in recent years.

Further, Non-patent Literature 3 reports on a case where high-strength bolts which were left air-exposed and subjected to corrosion for a certain period of time were collected and hydrogen concentrations occluded in the bolts were measured. Non-patent Literature 3 also reports on the results of investigating the behavior of hydrogen penetration caused by corrosion resulting from air exposure. The investigation employed an electrochemical hydrogen permeation method, in which test equipment for exposing one surface of a steel sheet to the external environment was used so as to detect the changes in anodic current value from the other surface side of the steel sheet, based on which the behavior of hydrogen penetration was investigated.

However, the data obtained by the air-exposure test disclosed in Non-patent Literature 3 is merely the test results all obtained under environmental factors associated with a specific geographical environment, and no consideration is given to continuously observing corrosion to be caused under various environments changing along with the traveling of the moving body.

Further, in the hydrogen permeation test on a steel sheet that has been air-exposed by using test equipment for exposing one surface of the steel sheet to the external environment, no consideration is given to changes in residual electric current on the anode side that accompany changes in environmental temperature, and thus the quantitativity of the measured values has also been under question.

As noted above, a steel material, which is widely used as an actual material, concerns the most at present as to the problem of delay fracture. However, it is pointed out the possibility that the problem of delay fracture similarly arises in other metallic materials in the future (see, for example, Non-patent Literature 4).

As described above, in the case of a moving body such as an automobile, its geographical environment changes along with the travelling thereof, and further, it may suffer a significant change of corrosive environment in accordance with physical factors (such as vibration, accumulation and falling of dust, splashes and drying of water and mud) applied thereto.

However, there has been found no example in which the amount of hydrogen penetrated due to corrosion is measured continuously and quantitatively in a moving body which is inevitably influenced by the aforementioned physical factors such as vibration and geographical change in environment.

In view of the aforementioned circumstances, the inventors of the present invention has first developed the following method, which is disclosed in Patent Literature 2 as "the method for measuring the amount of hydrogen penetrated into a metal accompanied by corrosion through electrochemical hydrogen permeation method, in which one side of a specimen is exposed to corrosive environment to be set to the penetration surface of hydrogen produced by corrosive reaction while the other side of the specimen is set to a hydrogen detection surface and, when the flux of hydrogen diffused to the hydrogen detection surface is measured as an anodic current in a state that the potential on the side of the hydrogen detection surface is held to -0.1 to +0.3V vs SCE, an electrochemical cell constituted of a group of a plurality of cells obtained by dividing the electrochemical cell at least in to two is arranged on the side of the hydrogen detection surface of the specimen, the cells in the cell group each being filled with an electrolyte aqueous solution having a pH of 9 to 13 and at least one of the cells serving as a base cell for compensating the residual electric current, a protective film for cutting off the contact with the corrosive environment is provided to a portion serving as the hydrogen penetration surface side in the base cell and the anodic current value detected in the cell other than the base cell is compensated by the residual electric current value detected in the base cell, to thereby calculate the amount of hydrogen penetrating from a corrosive surface on the basis of the compensated anodic current value", in accordance with the preamble of claim 1.

CITATION LIST

Patent Literature

> PTL 1: JP 2005-69815 A
> PTL 2: JP 2011-179893 A

Non-patent Literature

NPL 1:   "Matsuyama Shinsaku: Delayed Fracture, THE NIKKAN KOGYO SHIMBUN, LTD., Tokyo (1989)"
NPL 2:   "Takai et al.: Summary of Corrosion Engineering Symposium, Vol.170, pp.47-54 (2010)"
NPL 3:   "Ohmura et al.: TETSU TO HAGANE - JOURNAL OF THE IRON AND STEEL INSTITUTE OF JAPAN, Vol.91, No.5, p.42 (2005)"
NPL 4:   "Takatori et al.: TETSU TO HAGANE - JOURNAL OF THE IRON AND STEEL INSTITUTE OF JAPAN, Vol.78, No.5, p.149 (1992)"

NPL 5:   "M. A. V. Devanathan, Z. Stachurski: Proc. Roy. Soc. London, Ser. A, 270, 90 (1962)"

SUMMARY OF INVENTION

(Technical Problem)

The invention disclosed in Patent Literature 2 allows accurate measurement of the amount of hydrogen penetrated into a metal due to corrosion, in consideration of changes in the residual electric current on the anode side resulting from temperature changes in the environment.

The present invention has an object of providing an apparatus for measuring the amount of hydrogen penetrated into metal, which can be preferably applied to the "method of measuring the amount of hydrogen penetrated into metal" disclosed in Patent Literature 2.

(Solution to Problem)

Specifically, primary features of the present invention are as follows.

1. An apparatus for measuring an amount of hydrogen generated due to corrosion of a specimen formed of a metallic material and penetrated into the metallic material by using an electrochemical hydrogen permeation method, the specimen having one surface exposed to a corrosive environment so as to serve as a penetration surface of hydrogen generated by a corrosion reaction while having the other surface as a hydrogen detection surface, the apparatus comprising:

an electrochemical cell constituted of a group of a plurality of cells disposed on the hydrogen detection surface side, the cells in the cell group each being filled with an electrolyte aqueous solution having a pH of 9 to 13 and having a reference electrode and a counter electrode independently disposed therein, wherein at least one of the plurality of cells in the cell group is configured as a base cell for compensating a residual electric current, the base cell having a protective film formed on a region on the hydrogen penetration surface side corresponding to a hydrogen detection region thereof for the purpose of cutting off a contact with a corrosive environment, and wherein the apparatus is configured to compensate the anodic current values detected in other cells than the base cell (7a) based on the residual electric current value detected in the base cell (7a), and to calculate an amount of hydrogen penetrating from a corrosive surface side based on the compensated anodic current values; the apparatus **being characterized** in that the electrolyte aqueous solution contains dimethylsulfoxide or dimethylformamide added thereto for the purpose of antifreezing and/or in that the electrochemical cell (1) filled with the electrolyte aqueous solution has a gas body (11) disposed therein, the gas body (11) being arranged without coming into contact with the hydrogen, detection surface.

2. The apparatus for measuring an amount of hydrogen penetrated into a metal according to the aforementioned reference 1, in which the reference electrode employs an Ir/Ir oxide electrode.

(Advantageous Effect of Invention)

The apparatus of the present invention is capable of accurately detecting the amount of hydrogen penetrated into metal due to corrosion.

Further, the use of the apparatus of the present invention allows continuous monitoring of the amount of hydrogen penetrating into a metallic material, the hydrogen being generated due to corrosion occurring at each part of the metallic material constituting a moving body such as automobiles, marine vessels, and rail cars under corrosive environment to which the moving body is exposed during use thereof, to thereby provide information needed for determining whether or not the delayed fracture is to be caused by the amount of hydrogen to penetrate due to corrosion that is to occur under the actual use environment.

BRIEF DESCRIPTION OF DRAWINGS

The present invention will be further described below with reference to the accompanying drawings, wherein:

FIG. 1 is an explanatory view for illustrating a prior art electrochemical hydrogen permeation method;
FIG. 2 is a view schematically illustrating an example of a measuring apparatus of the prior art;
FIG. 3 is a view schematically illustrating reactions in a prior art cell on a corrosive surface (hydrogen penetration surface) side with no protective film formed thereon and on a hydrogen detection surface side;
FIG. 4 is a view illustrating an example where a gas body is disposed inside a cell according to the present invention;
FIG. 5 is a view illustrating another example where a gas body is disposed inside a cell according to the present invention;
FIG. 6 is a graph showing changes in temperature and humidity in a prior art Example;
FIG. 7 is a graph showing temporal change of an anodic current detected in each channel;
FIG. 8 is a picture showing external appearances of prior art experimented samples on the corrosive surface side;
FIG. 9 is a view schematically illustrating a prior art measurement system in which the measuring apparatus mounted on an automobile to measure an anodic current;
FIG. 10 is a graph showing differences in anodic current density detected at different positions of the automobile, in comparison between a case where compensation of the residual electric current was performed based on the reference electrode and a case where such compensation was not performed (Comparative Example);

FIG. 11 is a view schematically illustrating another example of the measuring apparatus of the present invention; and FIG. 12 is a graph showing the change in minimum temperature during the measurement period.

DESCRIPTION OF EMBODIMENTS

Although technology of the present invention is applicable to any self-movable moving body, including: various vehicles such as automobiles, autobicycles, and rail cars; marine vessels; and aircraft, an embodiment of the present invention is hereinafter described in detail by taking an automobile as a representative example. Further, a steel sheet is described herein as a representative example to which the present invention is applied, although a metallic material to be evaluated is not necessarily limited thereto.

An amount of hydrogen generated due to corrosion of a metallic material and penetrated into a metallic material is measured based on the measurement principle of electrochemical hydrogen permeation, in which a steel sheet surface on the hydrogen penetration surface side is exposed to a corrosion environment so that hydrogen generated due to corrosion penetrates into the steel, and the hydrogen thus penetrated is taken out from the other side, to thereby measure the amount of hydrogen penetrated.

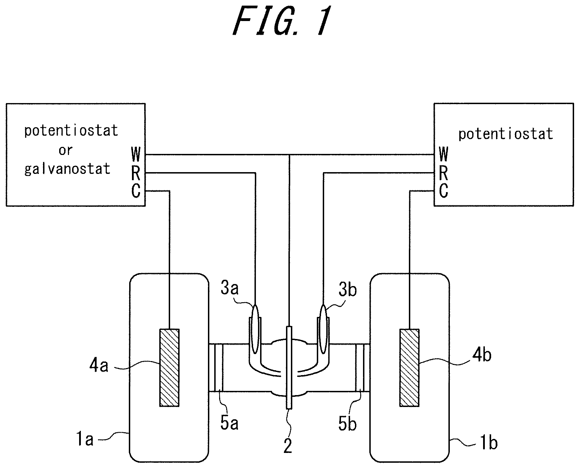

The electrochemical hydrogen permeation was developed by Devanathan and Stachurski in 1962 (Non-patent Literature 5), which uses two electrolysis cells 1 a, 1 b arranged opposite to each other across a sample 2, as schematically illustrated in FIG. 1. In FIG. 1, the sample surface on the electrolysis cell 1a side on the left is cathode-polarized at a constant potential or at a constant current so as to generate and charge hydrogen. The sample 2 is anode-polarized by the electrolysis cell 16 on the right at a constant potential so as to oxidize hydrogen permeated through the sample 2 into hydrogen ions, to thereby obtain the amount of hydrogen thus permeated based on the current value thereof. FIG. 1 also illustrates reference electrodes 3a, 3b, and electrodes 4a, 4b. In particular, the electrode 4b is specifically referred to as counter electrode or coefficient electrode. The electrode 4a is connected to a potentiostat for applying a constant potential or a galvanostat for applying a constant current, while the electrode 4b is connected to a potentiostat for applying a constant potential. Glass frits 5a, 5b are also provided for removing influence of gas and the like generated by the counter electrodes 4a, 4b.

The aforementioned electrochemical hydrogen permeation itself has conventionally been well-known as "a method of measuring a hydrogen diffusion coefficient in a steel sheet". The electrochemical hydrogen permeation is originally adapted to cathodize one side of a sample to electrolytically charge hydrogen, while anodizing the other side of the sample so as to take out the hydrogen therefrom, as illustrated in FIG. 1. A study on one of the applications of this technique has been reported in which a surface corresponding to the hydrogen-charging surface is exposed to a corrosive environment (Non-patent Literature 3). However, as described above, the measuring method disclosed in Non-patent literature 3 involves a problem in that no consideration is given to the change in measured current value resulting from temperature changes. Further, the anodic current to be measured on the hydrogen detection surface side in the electrochemical hydrogen permeation is superposed with a passive current of the sample material as well as the oxidation current of hydrogen. This passive current accounts for the most part of the residual electric current, and is influenced by various factors. In particular, the passive current greatly varies depending on the temperature.

The anodic current to be measured on the hydrogen detection surface side by the electrochemical hydrogen permeation method is weak, and thus, an accurate anodic current value cannot be obtained unless the temperature dependency of the residual electric current is compensated.

As a result of various studies to solve the aforementioned problems, the inventors of the present invention have achieved the followings. That is, the electrochemical cell to be provided on the hydrogen detecting surface side is formed of a group of a plurality of cells, obtained by dividing the cell on the same specimen into at least two, at least one of the cells serving as a base cell for compensating the residual electric current, and a protective film for cutting off a contact with a corrosion environment is provided on a region on the hydrogen penetration surface side corresponding to the hydrogen detection region of the base cell, to thereby compensate the temperature dependency of the residual electric current.

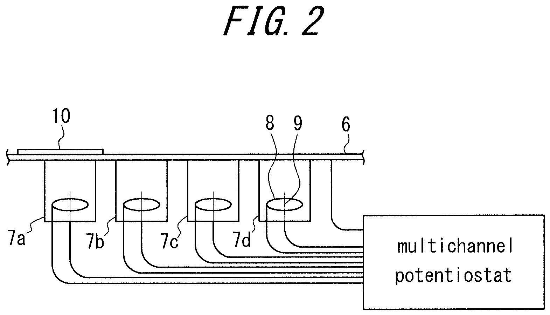

FIG. 2 schematically illustrates an example of a measuring apparatus of PTL. The example of FIG. 2 includes four cells 7a, 7b, 7c, and 7d on the hydrogen detection side of a steel sheet 6 as a specimen, in which the leftmost cell 7a is configured as the base cell for compensating the residual electric current. FIG. 2 also illustrates a counter electrode (Pt wire) 8, and a reference electrode (Ir wire) 9. At least two cells are required, preferably without exceeding a maximum of four because too many cells make the handling thereof complicated. In FIG. 2, the surface temperature of the steel sheet in each cell and the temperature of the electrolyte solution in each cell are set to the same temperatures. Further, a protective film 10 is provided on the hydrogen penetration surface side of the base cell 7a. The portion thus covered by the protective film 10 is exempted from corrosion, and thus no hydrogen penetrates thereinto. Therefore, the current to be measured on the hydrogen detection surface side of the base cell can be considered as the residual electric current itself.

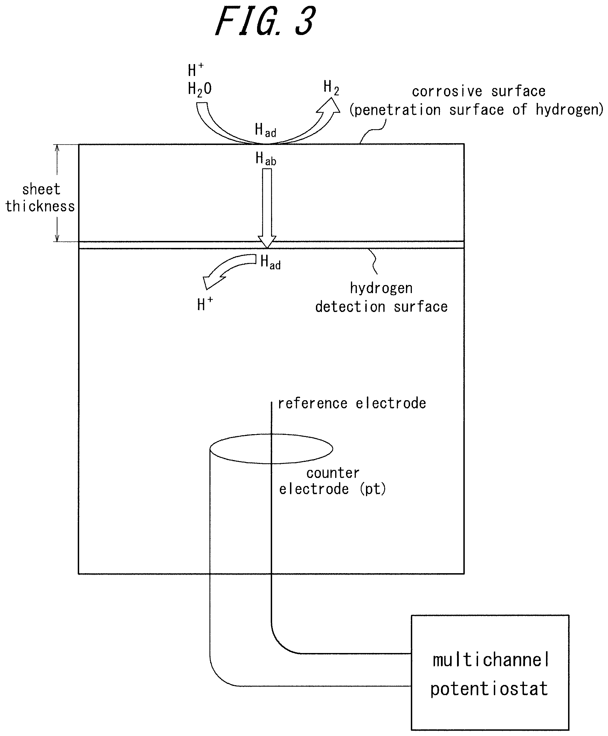

FIG. 3 schematically illustrates reactions to occur on the corrosive surface (hydrogen penetration surface) side, and on the hydrogen detection surface side in a cell with no protective film (also referred to as channel). The surface potential on the hydrogen detection surface side is retained at a potential sufficient enough to cause ionization reaction of hydrogen, so as to take out all the hydrogen reached to the detection surface side through diffusion, as hydrogen ions. The steel sheet surface on the hydrogen detection surface side is passivated, and therefore, the anodic current detected on the hydrogen detection side can be considered to substantially correspond to the hydrogen permeation current. Therefore, the current value thus obtained is compensated based on the residual electric current value obtained in the base cell, to thereby obtain an accurate anodic current value irrespective of the changes in residual electric current resulting from temperature changes, with the result that the amount of permeated hydrogen can be accurately calculated based on the anodic current value.

In order to retain the steel sheet on the hydrogen detection surface side in a passive state, the solution in the anode electrode chamber needs to be an electrolyte solution having a pH of 9 to 13. The reason is as follows. That is, with pH less than 9, the passive state of the steel surface is difficult to maintain at a predetermined potential, whereas with pH exceeding 13, there may be caused great damage to environment in the case of accidental leakage. NaOH solution of 0.1 to 0.2 M (mole/liter) is suitably employed as the electrolyte solution with proper pH. However, the electrolyte solution is not necessarily limited to NaOH solution of 0.1 to 0.2 M, and may be any electrolyte solution as long as capable of ensuring the passive state of the steel sheet surface when retaining the steel sheet surface on the hydrogen detection surface side at a potential sufficient enough to cause ionization reaction of hydrogen. Further, it is advantageous to use a gelled electrolyte in place of an electrolyte solution, in terms of preventing leakage as well as ease in handling.

Further, the hydrogen detection surface needs to be constantly retained at a potential ranged from -0.1 to +0.3V vs SCE. The reason is that the potential of the hydrogen detection surface falling out of the range fails to obtain a stable hydrogen ionization current. <::!J Here, SCE refers to a saturated calomel electrode, and the potential of SCE with respect to the standard hydrogen electrode (SHE) is obtained as +0.244V (vs SHE, 25°C).

As the reference electrode for controlling the potential, various electrodes currently available for practical use can be employed. <A However, in the case of using an electrode containing a chloride, such as Ag/ AgCl electrode, the solution in the anode electrode chamber may be contaminated by chloride ions, which may destroy the passive state on the sample surface to increase the residual electric current, possibly leading to inaccuracy in measured value.

Various studies have made on the reference electrode that can eliminate the aforementioned problems, and there have been found out that Ir wire immersed in the solution in the anode electrode chamber makes the reference electrode as Ir/Ir oxide electrode, which allows a stable current to be obtained over a long period. That is, the Ir/Ir oxide electrode is most preferred as the reference electrode, which can stably provide a potential of about -0.04V vs SSE. Here, SSE refers to a silver - silver chloride electrode, and the potential of SSE with respect to the standard hydrogen electrode (SHE) is obtained as +0.199V (vs SHE, 25°C).

Further, the surface of the hydrogen detection surface may preferably be covered with a metal that is high in hydrogen diffusion constant and capable of accelerating the oxidation reaction of hydrogen. Examples such a metal may include palladium (Pd), a palladium (Pd) alloy, and nickel (Ni). The hydrogen detection surface thus covered with such a metal or an alloy is capable of maintaining the residual electric current thereon at low value, and also capable of accelerating the oxidation reaction of penetrating hydrogen on the hydrogen detection surface, to thereby increase sensitivity to the anodic current obtained as a result of the ionization of hydrogen. Here, Pd is larger in hydrogen diffusion constant than Ni, and also has the advantage of reducing the residual electric current.

In coating the surface with Pd or a Pd alloy, the surface may be subjected to cathode electrolysis in an aqueous solution containing palladium ions such as $[Pd(NH_3)_4]Cl_2 \cdot H_2O$, to thereby form a plating on the surface. Pd-Ni alloy and Pd-Co alloy may be used as the Pd alloy. The Pd plating or the Pd alloy plating may preferably be 10 to 100 nm in film thickness.

In covering the surface with Ni, the surface may be subjected to cathode electrolysis in a known plating bath such as Watts bath, to thereby form a Ni plating on the surface. The Ni plating may also preferably be 10 to 100 nm in film thickness.

Further, a Pd or Pd-alloy plating may be formed on the Ni plating.

The protective film to be provided on the hydrogen penetration surface is not particularly limited, as long as capable of cutting off a corrosive environment. A specific example thereof may include a stainless foil attached to be pasted via an organic adhesive or the like.

As described above, according to the present invention, the amount of hydrogen penetrating into a metal due to corrosion can be accurately detected, irrespective of the changes in environment such as temperature changes.

Therefore, the measuring apparatus can be attached to a moving body such as an automobile, a marine vessel, and a rail car, so that the amount of hydrogen penetrating into the metallic material constituting the moving body can be accurately monitored continuously, irrespective of the change in environment to which each part of the metallic material is exposed during use.

As a result, a precise judgment can be made for each moving body as to whether or not the delayed fracture is to be caused by the amount of hydrogen to penetrate due to corrosion that is to occur under an actual use environment of the moving body.

In the meantime, it has also been found out, in the course of repeating various studies using the apparatus, that the solution may leak out from within the cell under conditions simulating a winter corrosive environment. The investigation of the cause of the solution leakage revealed that the solution inside the cell freezes and swells at low temperature, causing damage to the cell. For the accurate monitoring of the hydrogen penetrating into the metallic site of a moving body, which is the feature of the present invention, the amount of penetrating hydrogen needs to be stably measured even in the case of driving in winter.

In view of the above, the inventors of the present invention have made further studies on an apparatus capable of stably measuring the amount of hydrogen penetrating into a metal due to corrosion, without having any damage caused to the cell which is ascribable to the frozen solution within the cell even in winter. Under a driving environment in urban areas in general, the electrolyte solution is considered to be seldom cooled to -5°C or lower. Thus, it can be deemed sufficient to have the freezing temperature of the electrolyte solution equal to or lower than -5°C.

The result of studies made on a method of lowering the freezing temperature of the electrolyte solution to -5°C or lower has identified that an organic compound effective in reducing the freezing temperature needs to be added to the electrolyte aqueous solution. The organic compound added comprises dimethylsulfoxide (DMSO) or dimethylformamide (DMFA), which are polar solvents with low electrochemical activities.

A suitable content of the aforementioned organic compound is about 5 to 30 vol%. The freezing temperature further decreases along with the increase in added amount of the organic compound.

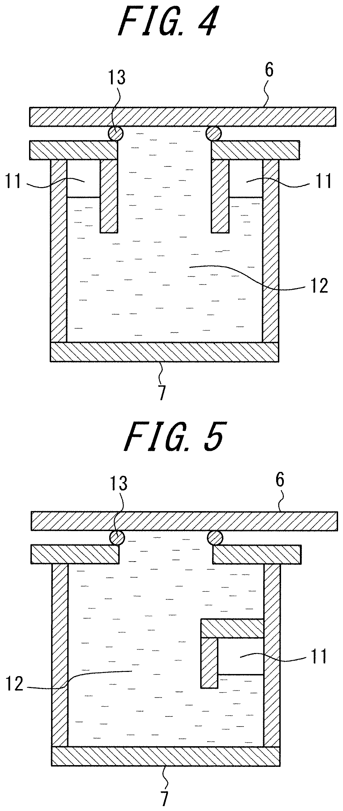

Further, it has been found effective to provide a gas body inside the cell in order to alleviate damage to the cell main body even in the case where the cell is exposed to an unanticipated low-temperature environment and has the electrolyte frozen, resulting in volume expansion of the electrolyte, because the gas body can contract to compensate for the volume expansion. However, even in this case, it is essential to dispose the gas body at a position without coming into contact with the hydrogen detection surface, because otherwise the hydrogen detection surface is deteriorated in sensitivity for hydrogen penetrating therethrough, which impairs the essential significance of the present invention. It is not specifically limited how to arrange the gas body without coming into contact with the hydrogen detection surface, and a sac or pouch filled with air may be disposed inside the cell, or the cell may be configured to have an internal structure as illustrated in FIGS. 4 and 5. Illustrated in FIGS. 4 and 5 are a gas body 11, an electrolyte 12, and an O-ring 13. Although the amount of the gas body is not particularly specified, it is preferred to have the gas body to 5 to 15% of the solution in volume fraction in view of the volume expansion resulting from water solidification. An inert gas may preferably used for the gas body, because an oxygen-containing gas body affects the anode reaction on the hydrogen detection surface.

EXAMPLES

Prior Art Example 1

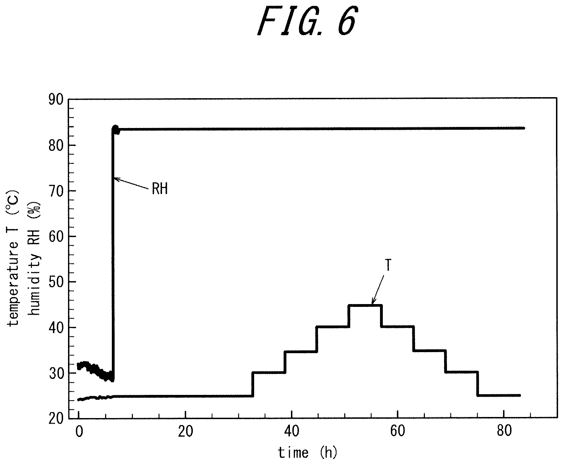

The measuring apparatus having four cells (CH1 to CH4) configured as illustrated in FIG. 2 was used to conduct an experiment. A soft steel sheet of 1.0 mm in thickness having a Pd plating formed on the hydrogen detection surface side to a thickness of 100 nm was used as a specimen. The channel 3 (CH3) served as the base cell, and a stainless foil was attached to be pasted, as

the protective film, to a portion serving as the corrosive surface side of CH3. On the surface on the corrosive surface side of each cell, 300 mL of 0.5M NaCl was dropped, and subsequently the surface was dried under conditions of 25°C and 35%RH (relative humidity) for at least 4 hours, which was then retained at 25°C and 85%RH (relative humidity) for at least 24 hours, before increasing the temperature stepwise. The hydrogen detection surface was retained at a potential of 0V vs SCE. Changes in temperature and humidity at this time are shown in FIG. 6.

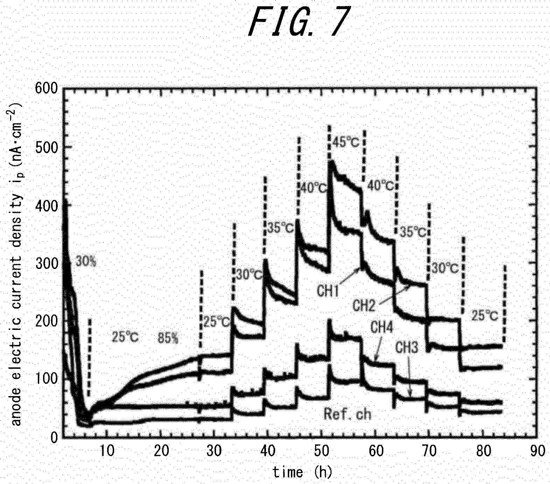

FIG. 7 shows changes in anodic current density detected in each channel correspondingly to the changes in temperature shown in FIG. 6.

It can be appreciated from FIG. 7 that the anodic current density value of the reference electrode (CH3), which was supposed to have no corrosion occurring on the steel sheet surface, also increased along with the increase in temperature. The increase of the anodic current density value can be considered ascribable to the fact that the residual electric current resulting from the oxidation current of Pd on the hydrogen detection surface side was increased due to the increase in temperature. Thus, the temperature dependency of the residual electric current is too large to ignore.

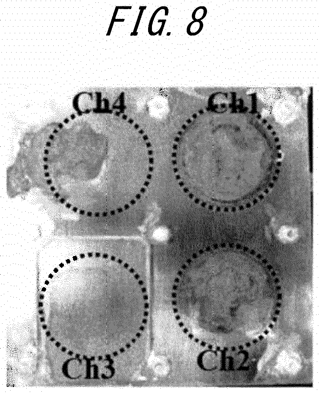

FIG. 8 is a picture showing the external appearances of the experimented samples on the corrosive surface side.

The anodic current density value of CH4 was smaller than those of the other CHs because 0.2M NaCl first dropped missed the point, and thus the corroded area on the hydrogen penetration surface side corresponding to the detection surface was smaller.

Therefore, the anodic current density value of the reference electrode (CH3) is subtracted from each of the anodic current density values obtained by CH1 and CH2, to thereby obtain accurate current density values of permeated hydrogen in the respective cells (CH1, CH2). The current density values of permeated hydrogen thus obtained may be averaged so as to obtain the current density value of hydrogen permeated through the steel sheet as a specimen.

Then, based on the current density value of permeated hydrogen obtained as described above, the amount of permeated hydrogen (amount of penetrated hydrogen) is calculated by the following equations.

In this manner, the current value of permeated hydrogen and also the amount of permeated hydrogen (amount of penetrated hydrogen) can both be accurately derived.

The current density value is converted into the amount of permeated hydrogen based on the following equations.

Current density of permeated hydrogen:

$$i_H \ (mA/cm^2 = 10^{-6} A/cm^2)$$

Amount of permeated hydrogen per unit area:

$M_H \ (mol/scm^2)$, $m_H \ (count/scm^2)$

$$M_H = i_H \times 1.036 \times 10^{-11} \ (mol/scm^2),$$

$$m_H = i_H \times 6.24 \times 10^{12} \ (count/scm^2)$$

Prior Art Example 2

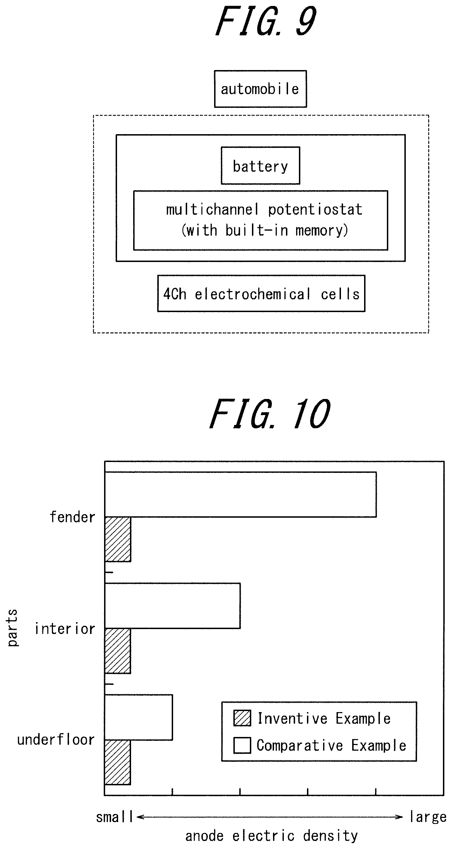

The measuring apparatus used in Prior Art Example 1 was actually mounted onto an automobile, to thereby construct a measurement system schematically illustrated in FIG. 9. The 4-channel cells were installed at three locations of (a) fender, (b) interior, and (c) underfloor (under surface of the floor). A multichannel potentiostat for driving by a battery was prepared, and stored in a trunk together with a dedicated battery. A soft steel sheet of 1.0 mm in thickness was similarly used as the sample material as in Example 1. The automobile was driven at an average of 40 km/h inside the premise of a steelworks for 6 hours from 9:00 to 15:00 every day of 5 days from Monday to Friday. The automobile was parked in a parking lot from 15:00 to 9:00 on the following day.

FIG. 10 shows the maximum values of the anodic current density detected during the aforementioned period, in comparison between the example in which the maximum value was compensated based on the reference electrode and the comparative example in which the maximum value was not compensated.

In the initial 5 days after setting the sample piece, there could hardly be seen any corrosion in each part, and as shown in FIG. 10, no difference could be found in anodic current density between the installation locations. In contrast, in Comparative Examples, there was found differences in anodic current density between the installation locations. The differences can be considered to result from a difference in temperature between a portion (fender) which underwent temperature increase due to daytime insolation and a portion (underfloor) which hardly saw any temperature increase.

It can be appreciated from the above that accurate anodic current density values (current density values of permeated hydrogen) can be obtained without being affected by changes in temperature, by compensating the measured anodic current density values based on the reference electrode. Example according to the present invention

A commercially-available soft steel sheet (of 0.8 mm in thickness) was used, which was subjected to shearing process so as to sized into 40 × 50 mm and polished on both surfaces to #2,000. Subsequently, in order to remove the processing layer formed during the polishing, the both surfaces were chemically polished to about 60 μm in thickness in an aqueous solution of a mixture solution of hydrofluoric acid and hydrogen peroxide solution.

(Plating on the hydrogen detection surface)

The hydrogen detection surface was Pd plated to about 100 mm in thickness, using a commercially-available K-pure palladium plating solution (manufactured by Kojima Chemicals Co., Ltd.).

(Electrolyte aqueous solution in the cells)

Sodium hydroxide aqueous solutions of 0.1 N containing dimethylsulfoxide (DMSO) at various ratios were used as the electrolyte aqueous solutions, and the freezing temperature in each case was measured.

(Configuration of the cell on the detection surface side)

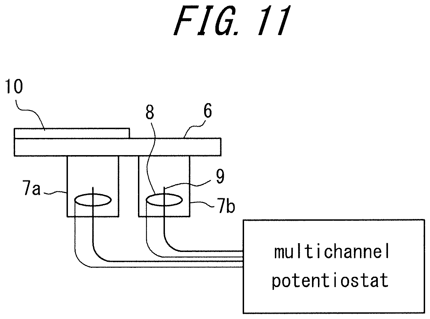

A measuring apparatus configured by including two cells illustrated in FIG. 11 was employed, in which portions corresponding to 7a and 7b were changed in configuration as follows.

Configuration A:

The configuration of FIG. 5 was employed with no gas body provided therein, and was filled with an electrolyte solution.

Configuration B:

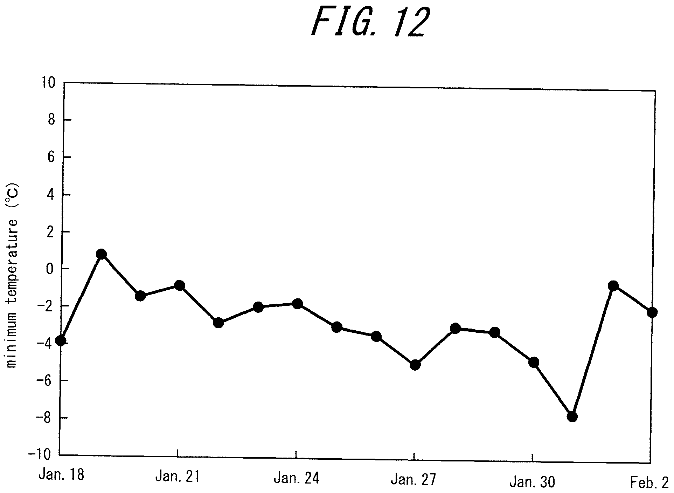

The configuration of FIG. 5 was employed, in which nitrogen was filled as the gas body to the amount of 15vol%.
As illustrated in FIG. 11, a steel sheet was installed on the cells configured as described above. An Ir/IrO$_x$ electrode was disposed as the reference electrode while a Pt wire was disposed as the counter electrode with the potential being set to 0V, and the cells were placed under a corrosive environment.
An epoxy resin and a stainless foil were arranged on one of the channels of the cells, so as to have one cell exempted from corrosion, in order to compensate changes in temperature.
The aforementioned cells were mounted on a commercially-available automobile, which was driven inside the premise of a steelworks for 15 days from January 18 to February 2, 2011. FIG. 12 shows changes in daily minimum temperature in the period obtained from the website of the Japan Meteorological Agency. In order to prevent leakage of the content fluid resulting from freezing, the driving was performed while being covered with a bag except for a steel sheet surface to be corroded.
Table 1 shows the date on which a cell fracture or leakage of the electrolyte solution was identified. Table 1 also shows maximum values of the current density obtained for each period, together with compensated maximum values of the current density obtained according to the present invention.
Further, after the elapse of the period, the cells of No. 2 and No. 4 were then left in a refrigeration tester of -20°C installed in a laboratory, and checked for fractures of the cells. Table 1 also shows the results thereof.

[Table 1]

| No. | Cell Structure | Content of Dimethyl Sulfoxide (vol%) | Freezing Temperature (°C) | Period I (Jan.18 to Jan. 22) | | Period II (Jan. 23 to Jan. 27) | | Period III (Jan. 28 to Feb. 2) | | Confirmed Date of Cell Fracture | Cell Fracture at -20°C | Remarks |
|---|---|---|---|---|---|---|---|---|---|---|---|---|
| | | | | Uncompensated Current Density ($nA/cm^2$) | Compensated Current Density ($nA/cm^2$) | Uncompensated Current Density ($nA/cm^2$) | Compensated Current Density ($nA/cm^2$) | Uncompensated Current Density ($nA/cm^2$) | Compensated Current Density ($nA/cm^2$) | | | |
| 1 | A | 0 | -0.3 | 18 | 3 | - | - | - | - | Jan. 27 | unadministered | Comparative Example |
| 2 | A | 10 | -5 | 19 | 3 | 42 | 29 | 43 | 33 | No fracture | identified | Inventive Example |
| 3 | A | 20 | -12 | 18 | 3 | 39 | 29 | 45 | 33 | No fracture | unadministered | Inventive Example |
| 4 | B | 10 | -5 | 17 | 3 | 40 | 30 | 42 | 34 | No fracture | No fracture | Inventive Example |

The followings are made apparent from the results shown in Table 1.

No. 1 is Comparative Example in which the electrolyte was formed only of a sodium hydroxide aqueous solution. Leakage of the electrolyte resulting from a fracture of the cell was identified on January 27 in Comparative Example of No. 1, whereas no fracture was identified in the cells of No. 2 to No. 4 as Inventive Examples.

Further, there was found hardly any evidence of corrosion on the steel sheet surface in Period 1, which means that there occurred no corrosion-induced hydrogen penetration into the steel sheet and thus no current value should be detected. However, it can be found that a relatively large current value was detected when the temperature compensation was not performed. The current value thus detected is considered ascribable to the residual electric current change caused by changes of temperature as described above, and it can be appreciated from Table 1 that the temperature changes can be eliminated through the temperature compensation according to Inventive Examples.

Further, as a result of comparing No. 2 to No. 4 having dimethylsulfoxide (DMSO) added thereto with No. 1 having no DMSO added thereto, there was found no difference in current value therebetween. Therefore, it can be appreciated that the measurement accuracy would not be affected by the addition of dimethylsulfoxide (DMSO).

Next, in Period 2 and Period 3, the automobile was driven on a wet road, and therefore corrosion of the steel sheet was identified, as well as the increase in current density that has correspondingly occurred. It can be appreciated that the amount of hydrogen generated along with the corrosion and penetrated into the steel sheet was monitored.

Further, a fracture was identified in No. 2 when retained in a low-temperature environment of -20°C. On the other hand, in the cell No. 4 having a gas body disposed therein, no fracture of the cell was identified even though the electrolyte was identified as frozen. The results confirmed that a gas body disposed within the cell was capable of suppressing fracture even with respect to unanticipated changes in temperature.

Industrial Applicability

The present invention enables continuous and accurate monitoring of the amount of hydrogen generated due to corrosion of a metallic material and penetrated into the metallic material, and thus the present invention can be suitably applied to a moving body in an ever-changing environment, where each part of the metallic material forming the moving body is exposed to a corrosive environment and corroded, and hydrogen is generated along with the corrosion and penetrates into the metallic material.

Reference Signs List

1 electrolysis cell

2 sample
3 reference electrode
4 electrode
4b counter electrode
5 glass frit
6 specimen (steel sheet)
7 cell
7a base cell
8 counter electrode
9 reference electrode
10 protective film
11 gas body
12 electrolyte
13 O-ring

**Claims**

**1.** An apparatus for measuring an amount of hydrogen generated due to corrosion of a specimen (6) formed of a metallic material and penetrated into the metallic material by using an electrochemical hydrogen permeation method, the specimen (6) having one surface exposed to a corrosive environment so as to serve as a penetration surface of hydrogen generated by a corrosion reaction while having the other surface as a hydrogen detection surface, the apparatus comprising:

an electrochemical cell constituted of a group of a plurality of cells (7a, 7b, 7c, 7d) disposed on the hydrogen detection surface side, the cells (7a, 7b, 7c, 7d) in the cell group each being filled with an electrolyte aqueous solution having a pH of 9 to 13 and having a reference electrode (9) and a counter electrode (8) independently disposed therein,

wherein at least one of the plurality of cells (7a, 7b, 7c, 7d) in the cell group is configured as a base cell (7a) for compensating a residual electric current, the base cell (7a) having a protective film (10) formed on a region on the hydrogen penetration surface side corresponding to a hydrogen detection region thereof for the purpose of cutting off a contact with a corrosive environment, and

wherein the apparatus is configured to compensate the anodic current values detected in other cells than the base cell (7a) based on the residual electric current value detected in the base cell (7a), and to calculate an amount of hydrogen penetrating from a corrosive surface side based on the compensated anodic current values; the apparatus being **characterized in that** the electrolyte aqueous solution contains dimethylsulfoxide or dimethylformamide added thereto for the purpose of antifreezing and/or

**in that** the electrochemical cell (1) filled with the electrolyte aqueous solution has a gas body (11) disposed therein, the gas body (11) being arranged without coming into contact with the hydrogen detection surface.

2. The apparatus for measuring an amount of hydrogen penetrated into a metal according to claim 1, wherein the reference electrode (9) employs an Ir/Ir oxide electrode.

**Patentansprüche**

1. Vorrichtung zum Messen einer Menge von Wasserstoff, das aufgrund der Korrosion eines aus einem Metallmaterial gebildeten Probestücks (6) erzeugt wurde und in das Metallmaterial durch Anwendung eines elektrochemischen Wasserstoffpermeationsverfahrens eindringt, wobei das Probestück (6) eine Oberfläche hat, die einer korrosiven Umgebung ausgesetzt ist, um so als eine Eindringungsfläche von Wasserstoff zu dienen, das durch eine Korrosionsreaktion erzeugt wird, während die andere Fläche als eine Wasserstofferkennungsfläche verwendet wird, wobei die Vorrichtung umfasst:

eine elektrochemische Zelle, die aus einer Gruppe einer Vielzahl von Zellen (7a, 7b, 7c, 7d) gebildet ist, die an der Seite der Wasserstofferkennungsfläche angeordnet sind, wobei die Zellen (7a, 7b, 7c, 7d) in der Zellengruppe jeweils mit einer wässrigen Elektrolytlösung mit einem pH-Wert von 9 bis 13 gefüllt sind und eine Bezugselektrode (9) und eine Gegenelektrode (8) haben, die darin unabhängig angeordnet sind,

wobei wenigstens eine aus der Vielzahl von Zellen (7a, 7b, 7c, 7d) in der Zellengruppe als eine Basiszelle (7a) konfiguriert ist, um einen elektrischen Reststrom zu kompensieren, wobei die Basiszelle (7a) einen Schutzfilm (10) hat, der an einem Bereich an der Seite der Wasserstoffeindringungsfläche entsprechend einem Wasserstofferkennungsbereich davon zum Zweck des Abschneidens eines Kontaktes mit einer korrosiven Umgebung ausgebildet ist, und

wobei die Vorrichtung konfiguriert ist, um die anodischen Stromwerte zu kompensieren, die in anderen Zellen als der Basiszelle (7a) auf der Basis des elektrischen Reststromwertes erfasst wurden, der in der Basiszelle (7a) erfasst wurde, und um eine Menge von Wasserstoff zu berechnen, die von einer korrosiven Flächenseite auf der Basis der kompensierten anodischen Stromwerte eindringt;

wobei die Vorrichtung **dadurch gekennzeichnet ist, dass** die wässrige Elektrolytlosung Dimethylsulfid und Demethylformamid enthält, die dort als Frostschutzmittel hinzugefügt wurden, und/oder dadurch, dass die mit der wässrigen Elektrolytlösung gefüllte elektrochemische Zelle (1) einen darin angeordneten Gaskörper (11) hat, wobei der Gaskörper (11) angeordnet ist, ohne mit der Wasserstofferkennungsfläche in Kontakt zu kommen.

2. Vorrichtung zum Messen einer Menge von in ein Metall eingedrungenem Wasserstoff nach Anspruch 1, wobei die Bezugselektrode (9) Ir/Ir-Oxidelektrode verwendet.

**Revendications**

1. Appareil permettant de mesurer une quantité d'hydrogène générée en raison de la corrosion d'un spécimen (6) formé d'un matériau métallique et pénétrant dans le matériau métallique en utilisant un procédé de perméation électrochimique de l'hydrogène, le spécimen (6) comportant une première surface exposée à un environnement corrosif de sorte à servir de surface de pénétration de l'hydrogène généré par une réaction de corrosion tout en ayant l'autre surface utilisée comme surface de détection de l'hydrogène, l'appareil comprenant :

une cellule électrochimique constituée d'un groupe d'une pluralité de cellules (7a, 7b, 7c, 7d) disposées sur le côté de la surface de détection de l'hydrogène, les cellules (7a, 7b, 7c, 7d) dans le groupe de cellules étant chacune remplie d'une solution aqueuse d'électrolyte présentant un pH de 9 à 13 et comportant une électrode de référence (9) et une contre électrode (8) disposées indépendamment à l'intérieur de celles-ci,

dans lequel l'une de la pluralité de cellules (7a, 7b, 7c, 7d) dans le groupe de cellules est configurée comme cellule de base (7a) afin de compenser un courant électrique résiduel, la cellule de base (7a) comportant un film protecteur (10) formé sur une zone du côté de la surface de pénétration d'hydrogène correspondant à une zone de détection de l'hydrogène dans le but de supprimer tout contact avec un environnement corrosif, et

dans lequel l'appareil est configuré pour compenser les valeurs de courant anodique détectées dans d'autres cellules que la cellule de base (7a) sur la base de la valeur du courant électrique résiduel détectée dans la cellule de base (7a), et pour calculer la quantité d'hydrogène pénétrant depuis le côté de surface de corrosion sur la base des valeurs de courant anodique compensées,

l'appareil étant **caractérisé en ce que** la solution aqueuse électrolyte contient du diméthylsulfoxyde ou du diméthylformamide ajouté à celle-ci, utilisé en tant qu'antigel, et/ou **en ce que** la cellule électrochimique (1) remplie de la solution aqueuse électrolyte comporte un corps de gaz (11) disposé en elle, le corps de gaz (11) étant agencé sans venir au contact de la surface de détection de l'hydrogène.

2. Appareil destiné à mesurer une quantité d'hydrogène pénétrant dans un métal selon la revendication 1, dans lequel l'électrode de référence (9) utilise une électrode d'oxyde Ir / Ir.

# FIG. 1

*FIG. 2*

# FIG. 3

*FIG. 4*

## FIG. 6

FIG. 7

FIG. 8

## FIG. 9

automobile

battery

multichannel potentiostat
(with built-in memory)

4Ch electrochemical cells

## FIG. 10

parts

fender

interior

underfloor

▨ Inventive Example
☐ Comparative Example

small ←――――――――――→ large
anode electric density

# FIG. 11

# FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005069815 A

- JP 2011179893 A

**Non-patent literature cited in the description**

- **MATSUYAMA SHINSAKU.** Delayed Fracture. THE NIKKAN KOGYO SHIMBUN, LTD, 1989
- **TAKAI et al.** *Summary of Corrosion Engineering Symposium,* 2010, vol. 170, 47-54
- **OHMURA et al.** *TETSU TO HAGANE - JOURNAL OF THE IRON AND STEEL INSTITUTE OF JAPAN,* 2005, vol. 91 (5), 42

- **TAKATORI et al.** *TETSU TO HAGANE - JOURNAL OF THE IRON AND STEEL INSTITUTE OF JAPAN,* 1992, vol. 78 (5), 149
- **M. A. V. DEVANATHAN ; Z. STACHURSKI.** *Proc. Roy. Soc. London,* 1962, vol. 270, 90